Europäisches Patentamt

European Patent Office

Office européen des brevets

(11) Veröffentlichungsnummer: **0 277 511 B1**

(12) **EUROPÄISCHE PATENTSCHRIFT**

(45) Veröffentlichungstag der Patentschrift: 11.12.91

(51) Int. Cl.⁵: **A61F 2/34**

(21) Anmeldenummer: 88100424.6

(22) Anmeldetag: 14.01.88

(54) **Hüftpfanne für eine Hüftgelenkendoprothese.**

(30) Priorität: 20.01.87 DE 3701381

(43) Veröffentlichungstag der Anmeldung:
10.08.88 Patentblatt 88/32

(45) Bekanntmachung des Hinweises auf die
Patenterteilung:
11.12.91 Patentblatt 91/50

(84) Benannte Vertragsstaaten:
AT BE CH DE ES FR GB IT LI LU NL

(56) Entgegenhaltungen:
EP-A- 0 139 356
WO-A-86/02261

(73) Patentinhaber: orthoplant Endoprothetik
GmbH
Leerkämpe 12
W-2800 Bremen 66(DE)

(72) Erfinder: Schelhas, Klaus-Dieter
Leerkämpe 12
W-2800 Bremen(DE)

(74) Vertreter: Eisenführ, Speiser & Strasse
Martinistrasse 24
W-2800 Bremen 1(DE)

**Beschreibung**

Die Erfindung betrifft eine Hüftpfanne für eine Hüftgelenk-Endoprothese, mit einer metallischen Aussenpfanne und mit einem Pfannenkörper aus Kunststoff in der Aussenpfanne, der stirnseitig eine freiliegende Pfannenfläche und in der Aussenpfanne eine umlaufende Stützfläche besitzt, wobei sich die Innenform der Aussenpfanne und die Stützfläche des Pfannenkörpers zum Scheitel hin verjüngen.

Eine derartige Hüftpfanne ist z.B. aus der EP-A-01 42 759 oder der EP-A-01 90 093 bekannt. Bei diesen bekannten Hüftpfannen ist die Aussenpfanne als Ring ausgebildet, dessen Innenform mindestens einen Kegelabschnitt enthält. Die Aussenform des Pfannenkörpers besitzt einen entsprechenden Kegelabschnitt und ist mittels einer Konus-Klemmverbindung in der Aussenpfanne fixiert. In der Pfannenschale des Pfannenkörpers, die an der weiten Stirnfläche der Aussenpfanne freiliegt, ist der Hüftkopf der zugehörigen Hüftprothese gelagert, und durch die erhebliche Beanspruchung des Pfannenkörpers durch den aufgrund der Beinbewegung bewegten Hüftkopf kommt es zum Kaltfliessen und einer anschliessenden Verformung des Kunststoff-Pfannenkörpers. Dadurch löst sich der Pfannenkörper mindestens partiell von der Aussenpfanne, und es kommt zu der unerwünschten dauernden Relativbewegung zwischen Pfannenkörper und Außenpfanne, die zu Abrieb des Kunststoffes und zu Entzündungen des angrenzenden natürlichen Gewebes führt.

Aus der WO-A-86 02 261 ist eine Hüftpfanne bekannt, bei der zwischen Außenpfanne und dem Pfannkörper eine Zwischenlage aus elastischem Material angeordnet ist. Diese Zwischenlage soll die über den Hüftkopf in die Pfanne eingetragenen Belastungen dämpfen oder ganz absorbieren und ermöglicht zu diesem Zweck eine vorgegebene begrenzte Relativbewegung zwischen dem Pfannenkörper und der Außenpfanne. Durch diese Relativbewegungen besteht die Gefahr einer schnellen Ermüdung und nachfolgenden Beschädigung der Prothese.

Aufgabe der Erfindung ist es demgegenüber, eine Hüftpfanne der eingangs genannten Art derart weiterzubilden, daß der Pfannenkörper möglichst großflächig und fest in der Außenpfanne fixierbar ist, so daß Relativbewegungen des Pfannenkörpers sowie der resultierende unvermeidliche Abrieb und/oder bleibende Verformung bei der Langzeitbenutzung vermieden werden.

Diese Aufgabe wird bei der Hüftpfanne der eingangs genannten Art erfindungsgemäß dadurch gelöst, daß die Stützfläche des Pfannenkörpers mittels mindestens eines Spannelementes unter Flächenpressung von einem metallischen Mantel eng anliegend eingefaßt ist, wobei des Spannelement einerseits am Pfannenkörper und andererseits am metallischen Mantel angreift, und daß der aus Pfannenkörper und Mantel gebildete Einsatz mit mindestens einem Befestigungselement eng anliegend in der Außenpfanne verankerbar ist.

Die Vorteile der Erfindung liegen insbesondere darin, daß der Pfannenkörper fest und unbeweglich in einem metallischen Mantel eingepreßt ist und zusammen mit dem metallischen Mantel einen Einsatz bildet, der eng anliegend und unbeweglich in der Außenpfanne verankert ist. Unerwünschte Relativbewegungen zwischen dem Kunststoff-Pfannenkörper und dem Mantel bzw. zwischen dem Mantel und der Außenpfanne werden dadurch vermieden. Der Arzt braucht während der Operation nur den aus Pfannenkörper, Mantel und Spannelement bestehenden Einsatz in die Außenpfanne einzusetzen und dann diesen Einsatz z. B. mittels eines Sprengringes in der Außenpfanne zu verankern. Durch die Formanpassung zwischen Pfannenkörper und Mantel sowie zwischen Mantel und der Aussenpfanne ist sichergestellt, dass vom Hüftkopf eingeleitete Kräfte grossflächig auf die Aussenpfanne und von dort in das Beckengewebe geleitet werden.

Es stellt einen grossen Vorteil der vorliegenden Erfindung dar, dass das Einsetzen des Pfannenkörpers in den Metallmantel vom Hersteller der Hüftpfanne - und nicht intraoperativ vom Arzt - durchgeführt wird, weil sich dabei auch kompliziertere Verankerungsmechanismen zwischen den beiden Teilen sicher einsetzen lassen, die während der Operation vom Arzt nicht eingesetzt werden können. Vorteilhafterweise wird z.B. der Pfannenkörper mitteis eines Aussengewindes in ein entsprechendes Innengewinde des Mantels fest eingeschraubt. Besonders bevorzugt werden weitere Spannelemente eingesetzt, mit denen der Pfannenkörper unter Erzeugung einer Flächenpressung in den Mantel gepresst werden, damit die Relativbewegungen und Verformungen des Pfannenkörpers sicher vermieden werden können.

Als Spannelement lässt sich ein Gewindering einsetzen, der in ein Innengewinde des Mantels eingeschraubt werden kann und bei seiner Schraubbewegung gegen die Pfannenfläche des Pfannenkörpers anläuft und diese in den topfförmigen Mantel so hineinpresst, dass die Stützfläche des Pfannenkörpers eng an der Innenfläche des Mantels anliegt.

Besonders bevorzugt ist das Innengewinde im Mantel, in welches der Pfannenkörper eingeschraubt wird, und das Innengewinde für den Gewindering, welches den Pfannenkörper in den Mantel hineinpresst, gegenläufig. Radiale Kraftkomponenten, die beim Bewegungsspiel des Hüftkopfes in der Hüftpfanne unvermeidlich sind, werden dann immer bewirken, dass das eine der beiden Gewinde unter diesem Bewegungsspiel fester verschraubt wird, während das andere Gewinde sich zu lockern trachtet. Ein Herauslösen des Pfannenkörpers aus dem Mantel wird dadurch sicher verhindert.

Um die Aussenpfanne mittels eines geeigneten Werkzeuges einfach in die zuvor präparierte Acetabulumhöhle einsetzen zu können, ist an ihrem Scheitel zentral eine Mehrkantöffnung eingearbeitet. Der Mantel besitzt an seinem Scheitel einen entsprechenden Mehrkant, bevorzugt einen Achtkant, um den aus Pfannenkörper und Mantel bestehenden Einsatz gegen Rotation zusätzlich zu sichern, und um insbesondere den Einsatz in verschiedenen relativen Winkelstellungen in die Aussenpfanne einsetzen zu können.

Dieses Merkmal ist besonders für Dysplasie-Pfannenkörper wichtig, deren Pfannenschale gegen die Rotationsachse der Aussenpfanne unter einem bestimmten Winkel verläuft, wobei der Rand der Pfannenschale unter demselben Winkel gegen die Umfangskante der Aussenpfanne geneigt verläuft. In denjenigen Fällen, in denen der Einsatz einer Dysplasie-Hüftpfanne angezeigt ist, kann dann der Arzt aufgrund der Mehrkant-Passung am Scheitel der Hüftpfanne zuerst die Aussenpfanne im Bekkenknochen einsetzen und anschliessend den Einsatz in der gewünschten Winkelstellung in die Aussenpfanne einsetzen und dann dort verankern.

Die Aussenpfanne ragt bevorzugt an der Pfannenöffnung axial über die Umfangskante des Mantels hinaus und besitzt eine innenliegende Umfangsnut, in die zur Verankerung des Einsatzes ein Sprengring vom Arzt einsetzbar ist. Der Sprengring überragt radial den Mantel und bevorzugt auch noch den Gewindering und stellt damit eine weitere Sicherung dar, die ein Lösen des Gewinderinges und/oder des Pfannenkörpers verhindert.

Die Innenform der Aussenpfanne und die formangepasste Aussenform des Mantels besitzen je einen Kegelabschnitt, der an seinem weiten Ende in einen offenen Zylinderabschnitt übergeht. Das Innengewinde zur Aufnahme des Gewinderinges sitzt in diesem äusseren Zylinderabschnitt, das Innengewinde zur Aufnahme des Pfannenkörpers ist bevorzugt im Kegelabschnitt angebracht. Die Umfangsnut zur Aufnahme des Sprengringes sitzt im Zylinderabschnitt der Aussenpfanne.

Besonders bevorzugt besitzt der Mantel an seinem Scheitel eine ebene Scheitelfläche, welche an den Kegelabschnitt angrenzt. Die Scheitelfläche ist als Mehrkant ausgebildet, der beim Einsetzen des Einsatzes eine Führung mit der Mehrkantöffnung der Aussenpfanne bildet und von dieser aufgenommen wird. Der Pfannenkörper besitzt dann die Aussenform eines Kegelabschnitts, die eng in der entsprechenden Innenform des Mantels zur Anlage kommt.

Der Kegelwinkel des Mantels bzw. der Innenform der Aussenpfanne ist dabei ausreichend gross, so dass sich der Einsatz klemmfrei in die Aussenpfanne einsetzen lässt, d.h., dass zwischen Aussenpfanne und Einsatz eine Klemmkonus-Wirkung zuverlässig verhindert wird.

Die Erfindung ermöglicht es, für verschieden grosse Aussenpfannen identische oder verschieden grosse Einsätze zu verwenden. Ausserdem lässt sich bei evtl. notwendigen Reoperationen lediglich der Einsatz der Hüftpfanne auswechseln, wenn die Aussenpfanne sicher verankert ist.

Gemäss einer besonders bevorzugten Ausführungsform der Erfindung ist das Befestigungselement zum Verankern des Einsatzes in der Aussenpfanne als Sicherungsring ausgebildet, der ein Aussengewinde besitzt. Die Aussenpfanne ist von der Umfangskante ausgehend mit einem entsprechenden innenliegenden Sicherungsgewinde versehen, welches den Einsatz in Richtung der Rotationsachse um etwa die Höhe des Sicherungsringes überragt. Wird der Sicherungsring in das Sicherungsgewinde eingeschraubt, so kommt der Sicherungsring schliesslich flächig gegen den Einsatz zur Anlage und wird in dieser Position fest gegen den Einsatz verschraubt. Bevorzugt enthält der Sicherungsring auch noch ein Innengewinde, welches vorteilhafterweise gegenläufig zu dem Aussengewinde läuft. Der Pfannenkörper besitzt an der zylindrischen Basis der Ringnut eine auswärts gerichtete Umfangslippe, welche in ihrer radialen Erstreckung geringfügig grösser als die Gewindetiefe des Innengewindes des Sicherungsringes ist. Wird der Sicherungsring bei dieser Ausführungsform der Erfindung gegen den Einsatz verschraubt, so schraubt sich das Innengewinde über die Umfangslippe und presst diese unter bleibender Verformung in die Gewindegänge. Durch die Verformung dieser Umfangslippe ist der Sicherungsring gegen Lockerung gesichert. Diese bleibende Fixierung des Sicherungsringes ist dann besonders wirksam, wenn das Aussengewinde und das Innengewinde des Sicherungsringes gegenläufig sind, wenn also z.B. das Aussengewinde rechtsläufig, und das Innengewinde linksläufig sind. Eine Lockerung des Sicherungsringes ist dann nur bei Zerstörung des im Bereich der Umfangslippe bleibend in den Pfannenkörper eingeformten Gegengewindes möglich.

Vorteilhafte Weiterbildungen der Erfindung sind durch die Merkmale der Unteransprüche gekennzeichnet.

Im folgenden wird ein Ausführungsbeispiel der Erfindung anhand der Zeichnung näher erläutert.

Es zeigen:

Fig. 1    eine Explosionsdarstellung eines aus Mantel und Pfannenkörper bestehenden Einsatzes im Schnitt;

Fig. 2 einen Schnitt durch die Aussenpfanne, in welche der Einsatz gemäss Fig. 1 einsetzbar ist;

Fig. 3 einen Schnitt durch die zusammengesetzte Hüftpfanne;

Fig. 4 einer der Einzelheit A der Fig. 1 entsprechende Ansicht einer weiteren Ausführungsform des Pfannenkörpers; und

Fig. 5 einen vergrösserten Schnitt durch eine zusammengesetzte Hüftpfanne, in welcher der Pfannenkörper gemäss Fig. 4 eingesetzt ist.

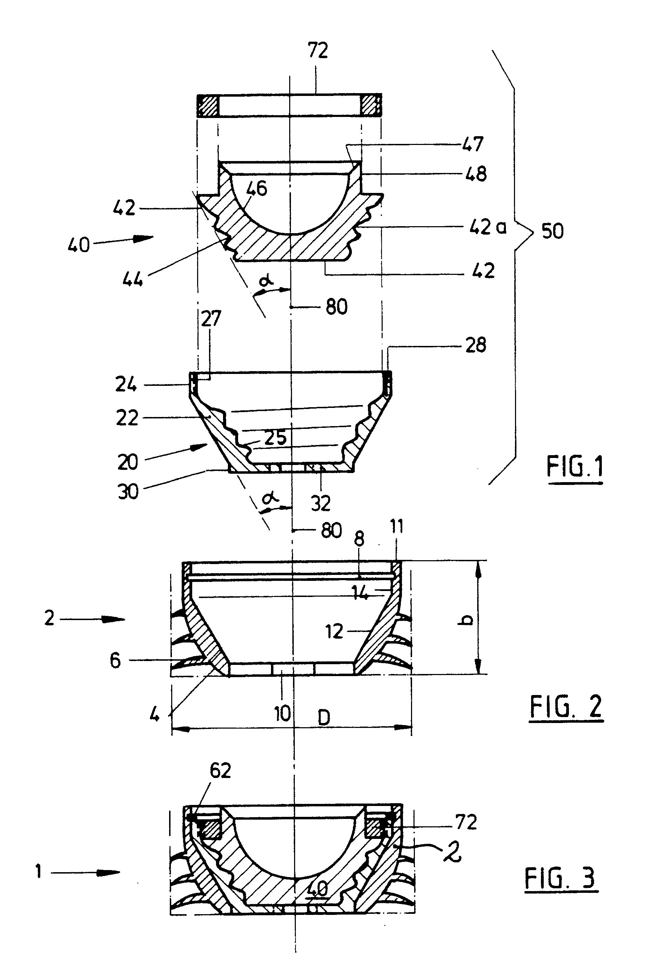

Fig. 1 zeigt eine Explosionsdarstellung des Einsatzes 50, der aus einem Mantel 20, einem Pfannenkörper 40 und einem Gewindering 72 besteht. Der Einsatz 50 wird in zusammengebautem Zustand in eine Aussenpfanne 2, vgl. Fig. 2, eingesetzt und dort mittels eines Befestigungselements, namlich eines Sprengringes 62, verankert und bildet somit die Hüftpfanne 1, vgl. Fig. 3. Der Pfannenkörper ist ein Formteil aus Kunststoff, in welches stirnseitig eine etwa halbkugelförmige Pfannenfläche 46 eingeformt ist, die stirnseitig in eine umlaufende Pfannenkante 47 ausläuft. An die Pfannenkante 47 schliesst eine Ringnut 48 an, die in eine zur Achse 80 rotationssymmetrische Stützfläche 42 übergeht und die Aussenform des Pfannenkörpers 40 bildet. Die Stützfläche besitzt die Gestalt eines zur Achse 80 rotationssymmetrischen Kegelabschnitts 42a, der eine ebene Scheitelfläche aufweist. In die Stützfläche 42 ist ein Aussengewinde 44 eingearbeitet.

Der Mantel 20 besteht aus Metall und besitzt die Form eines kegelstumpfförmigen Topfes, der ebenfalls eine ebene Scheitelfläche 32 besitzt und die Stützfläche 42 des Pfannenkörpers 40 eng anliegend einfasst. An dem weiten Ende des Kegelabschnitts 22 schliesst sich ein offener Zylinderabschnitt 24 an, der ein Innengewinde 27 enthält. Die Innenfläche des Kegelabschnitts 22 des Mantels 20 besitzt ein Innengewinde 25, in welches das Aussengewinde 44 des Pfannenkörpers 40 einschraubbar ist. Der eingeschraubte Pfannenkörper 40 liegt dann mit seiner Stützfläche 42 eng an der Innenfläche des Gesamtmantels 20 an. Zusätzlich wird ein Gewindering 72 in die Ringnut 48 des Pfannenkörpers 40 eingeführt und im angrenzenden Innengewinde 27 des Mantels 20 so fixiert, dass der Gewindering 72 den Pfannenkörper unter Flächenpressung in den Mantel 20 hineinpresst und dort hält und gegen Verdrehen sichert, weil das Innengewinde 27 zum Innengewinde 25 gegenläufig ausgebildet ist.

Die Aussenpfanne 2 aus Metall besitzt die Form eines Ringes, dessen Innenform einen Kegelabschnitt 12 besitzt, an dessen engem Ende eine Mehrkantöffnung 10 zentral zur Ringachse 80 angeformt ist und dessen weites Ende in einen Zylinderabschnitt 14 übergeht, der mit einer stirnseitigen Umfangskante 11 endet. Im Zylinderabschnitt 14 ist - in vorgegebenem Abstand von der Umfangskante 11 - eine Umfangsnut 8 eingearbeitet. Der Kegelwinkel $\alpha$ des Kegelabschnitts 12 ist mit dem Kegelwinkel $\alpha$ des Mantels 20 identisch. Die Aussenform der Aussenpfanne 2 geht von dem Zylinderabschnitt zum Scheitel hin in einen Kugelabschnitt 4 über, der ein zur Kegelachse 80 umlaufendes Aussengewinde 6 trägt, welches als Schneidgewinde ausgebildet ist und über die gesamte axiale Länge b der Aussenpfanne einen etwa konstanten Aussendurchmesser D aufweist. Der Einsatz 50 wird herstellerseitig zusammengebaut, und zwar wird der Pfannenkörper 40 in den Mantel 20 eingeschraubt und dann mit dem Gewinde 72 unter Flächenpressung gesichert. Während der Operation wird vom Operateur der Einsatz 50 in die Aussenpfanne 2 eingesetzt, wobei der Mehrkant 30 am Scheitel 32 des Mantels in die Mehrkantöffnung 10 hineinragt. Anschliessend wird der Einsatz 50 mit einem Sprengring 62 gesichert, der vom Operateur in die Umfangsnut 8 eingesetzt wird, und der den Mantel 20 und den Gewindering 72 radial überragt.

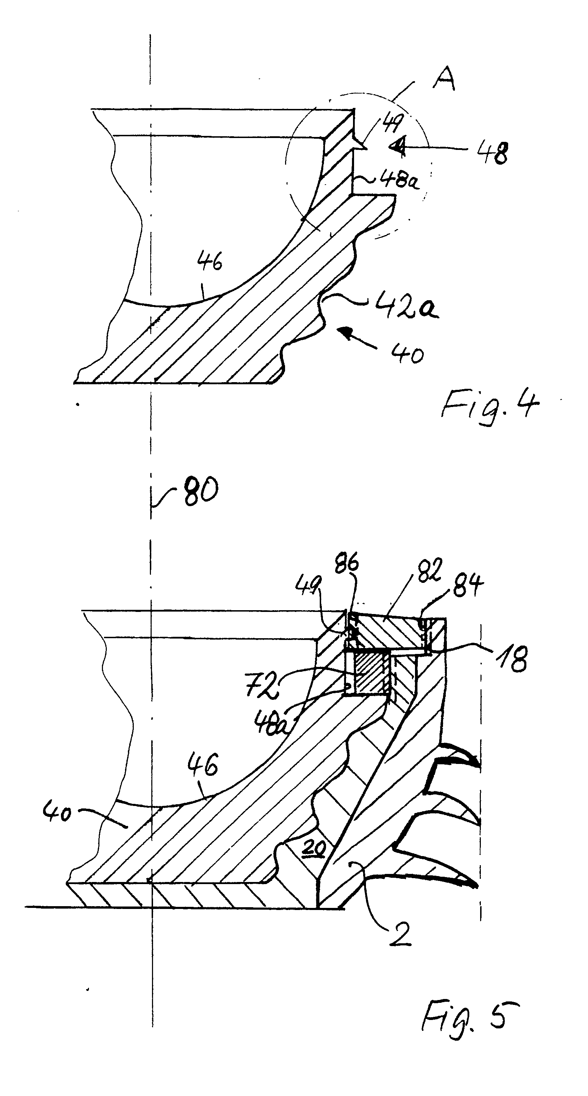

Fig. 4 zeigt eine der Einzelheit A der Fig. 1 entsprechende Darstellung einer zweiten Ausführungsform des Pfannenkörpers, welcher in einer Hüftpfanne gemäss Fig. 5 Verwendung findet. Die Grundform des Pfannenkörpers 40 der Fig. 4 entspricht in allen Einzelheiten dem Pfannenkörper gemäss Fig. 1. Zusätzlich ist jedoch an der zylindrischen Basis 48a der Ringnut 48 in einer Höhe, die etwas grösser als die Dicke des Gewinderinges 72 ist, eine radial auswärts gerichtete Umfangslippe 49 angeformt.

Die Hüftpfanne gemäss Fig. 5 unterscheidet sich von der Hüftpfanne gemäss Fig. 3 dadurch, dass statt des Sprengringes 62 ein Sicherungsring 82 verwendet wird, um den Einsatz 50 in der Aussenpfanne 2 zu verankern. Der Sicherungsring 82 besitzt ein Aussengewinde 84, welches in ein entsprechendes Sicherungsgewinde 18 der Aussenpfanne 2 eingreift. Das Sicherungsgewinde 18 erstreckt sich von der Umfangskante 11 auf der Innenfläche der Aussenpfanne 2 in Richtung der Rotationsachse 80 bis unterhalb der Oberkante des Einsatzes 50 und besitzt einen grösseren Durchmesser als der Einsatz 50 an seinem der Umfangskante 11 zugewandten Ende. Der Sicherungsring 82 erstreckt sich mit seiner radial innenliegenden Innenfläche bis hin zu der zylindrischen Basis 48a der Ringnut 48 des Pfannenkörpers 40. Der Sicherungsring 82 besitzt an seiner Innenfläche ein Innengewinde 86, welches gegenläufig zu seinem Aussengewinde 84 ist. Nachdem der Einsatz 50 in die Aussenpfanne 2 eingeschraubt ist, wird anschliessend der Sicherungsring 82 in das Sicherungsgewinde 18 der Aussenpfanne 2 geschraubt, bis die Unterseite des Sicherungsringes 82 flächig gegen den Gewindering 72 des Einsatzes 50 anliegt und den Einsatz 50 fest in den Aussenring 2 presst. Beim Einschrauben des Sicherungsringes schraubt sich sein Innengewinde 86 über die Umfangslippe 49 und presst diese - unter bleibender Verformung - in das Innengewinde 86. Die Höhe des Sicherungsringes 82 ist so bemessen, dass der Sicherungsring 82 in seiner eingeschraubten Verankerungsposition etwa bündig mit der Umfangskante 11 der Aussenpfanne 2 abschliesst. Der Gewindering 72 zur Befestigung des Pfannenkörpers 40 in dem Mantel 20 besitzt bei dieser Ausführungsform der Erfindung einen Innendurchmesser, der grösser als der Aussendurchmesser der Ringnut 48 ist, damit der Gewindering 72 bei seiner Montage über die Umfangslippe 49 geschoben werden kann, ohne die Umfangslippe 49 zu verformen.

**Patentansprüche**

1. Hüftpfanne (1) für eine Hüftgelenk-Endoprothese, mit einer metallischen Außenpfanne (2) und mit einem Pfannenkörper (40) aus Kunststoff in der Außenpfanne (2), der stirnseitig eine freiliegende Pfannenfläche (46) und in der Außenpfanne (2) eine umlaufende Stützfläche (12) besitzt, wobei sich die Innenform der Außenpfanne (2) und die Stützfläche (42) des Pfannenkörpers (40) zum Scheitel hin verjüngen, dadurch gekennzeichnet, daß die Stützfläche des Pfannenkörpers (40) mittels mindestens eines Spannelementes (72) unter Flächenpressung von einem metallischen Mantel (20) eng anliegend eingefaßt ist, wobei des Spannelement (72) einerseits am Pfannenkörper (40) und andererseits am metallischen Mantel (20) angreift, und daß der aus Pfannenkörper (40) und Mantel (20) gebildete Einsatz (50) mit mindestens einem Befestigungselement (62, 82) eng anliegend in der Außenpfanne (2) verankerbar ist.

2. Hüftpfanne nach Anspruch 1, dadurch gekennzeichnet, daß der Pfannenkörper (40) an seiner Stützfläche (42) ein Außengewinde (44) besitzt, das in einem entsprechenden Innengewinde (25) des Mantels (20) fest verschraubbar ist.

3. Hüftpfanne nach Anspruch 1 oder 2, dadurch gekennzeichnet, daß die Spannelemente einen Gewindering (72) enthalten, der an der der Pfannenfläche (46) benachbarten Umfangskante in ein Innengewinde (27) des Mantels (20) einschraubbar ist und bei seiner Verschraubung gegen den Pfannenkörper (40) drückt.

4. Hüftpfanne nach einem der Ansprüche 1 bis 3, dadurch gekennzeichnet, daß das Innengewinde (25) für den Pfannenkörper (40) und das Innengewinde (27) für den Gewindering (72) gegenläufig sind.

5. Hüftpfanne nach einem der vorstehenden Ansprüche, dadurch gekennzeichnet, daß der Mantel (20) am Scheitel des Pfannenkörpers (40) geschlossen ist und einen auswärts gerichteten Mehrkant (30) besitzt, der in eine entsprechende Mehrkantöffnung (10) am Scheitel der Außenpfanne (2) einsteckbar ist.

6. Hüftpfanne nach einem der vorstehenden Ansprüche, dadurch gekennzeichnet, daß die Befestigungselemente (62; 82) einen Sprengring (62) enthalten, der in einer Umfangsnut (8) der Außenpfanne (2) lösbar einsetzbar ist und den Einsatz (50) radial überragt.

7. Hüftpfanne nach Anspruch 6, dadurch gekennzeichnet, daß der Sprengring (62) die Umfangskante (28) des Mantels (20) und den Gewindering (72) zwischen Mantel (20) und Pfannenkörper (40) radial überragt.

8. Hüftpfanne nach einem der Ansprüche 1 bis 5, dadurch gekennzeichnet, daß die Befestigungselemente (62, 82) einen Sicherungsring (82) mit einem Außengewinde (84) enthalten, daß die Außenpfanne (2) von der Umfangskante (11) ausgehend ein entsprechendes innenliegendes Sicherungsgewinde (18) besitzt, welches den Einsatz (50) in Richtung der Achse (80) überragt, und daß der Sicherungsring (82) in dem Sicherungsgewinde (18) fest gegen den Einsatz (50) verschraubt wird.

9. Hüftpfanne nach Anspruch 7, dadurch gekennzeichnet, daß der Sicherungsring (82) in seiner Endlage flächig gegen den Gewindering (72) des Einsatzes (50) anliegt.

10. Hüftpfanne nach Anspruch 8, dadurch gekennzeichnet, daß der Sicherungsring (82) ein Innengewinde (86) aufweist, welches bei Einschrauben des Sicherungsringes (82) in die Außenpfanne (2) eine radial auswärts gerichtete Umfangslippe aufweist und unter bleibender Verformung in die Gewindegänge preßt.

**11.** Hüftpfanne nach Anspruch 10,
dadurch gekennzeichnet, daß das Außengewinde (84) und das Innengewinde (86) des Sicherungsringes gegenläufig sind.

**12.** Hüftpfanne nach einem der vorstehenden Ansprüche,
dadurch gekennzeichnet, daß die Innenform der Außenpfanne (2) und die Außenform des Mantels (20) einen Kegelabschnitt (12 bzw. 22) besitzen, der an seinem weiten Ende in einen offenen Zylinderabschnitt (14 bzw. 24) übergeht.

**13.** Hüftpfanne nach Anspruch 12,
dadurch gekennzeichnet, daß die Außenpfanne (2) an dem engen Ende des Kegelabschnitts (12) in die Mehrkantöffnung (10) übergeht, und daß der entsprechende Mehrkant (30) des Mantels (20) am engen Ende des Kegelabschnitts (22) an einer ebenen Scheitelfläche sitzt.

**14.** Hüftpfanne nach einem der Ansprüche 12 oder 13,
dadurch gekennzeichnet, daß das Innengewinde (24) des Mantels (20) um die Kegelachse (80) umlaufend am Kegelabschnitt (22) angeordnet ist, und daß die Stützfläche (42) des Pfannenkörpers (40) einen entsprechenden Kegelabschnitt (42a) aufweist, der das entsprechende Außengewinde (44) trägt.

**15.** Hüftpfanne nach einem der vorstehenden Ansprüche,
dadurch gekennzeichnet, daß der Einsatz (50) aus Mantel (20) und Pfannenkörper (40) klemmfrei in der Aussenpfanne (2) angeordnet ist.

**16.** Hüftpfanne nach einem der vorstehenden Ansprüche,
dadurch gekennzeichnet, daß der Mantel (20) eine konstante Wandstärke besitzt.

## Claims

**1.** A hip cup (1) for a hipjoint endoprosthesis, having a metal outer cup (2) and in the outer cup (2) a cup body (40) of plastics which at the endface has an exposed cup area (46) and in the outer cup (2) a circumferential bearing area (12), whilst the shape of the inside of the outer cup (2) and the bearing area (42) of the cup body (40) taper in towards the apex,
characterized in that the bearing area of the cup body (40) is framed by means of at least one clamping member (72) in close contact under surface pressure from a metal jacket (20), the clamping member (72) engaging at one side in the cup body (40) and at the other in the metal jacket (20), and that the insert (50) formed of the cup body (40) and the jacket (20) my be anchored in close contact with the outer cup (2) by at least one fastener (62, 82).

**2.** A hip cup as in Claim 1,
characterized in that the cup body (40) on its bearing area (42) has a male thread (44) which may be screwed firmly into a corresponding female thread (25) in the jacket (20).

**3.** A hip cup as in Claim 1 or 2,
characterized in that the clamping members include a threaded ring (72) which at the peripheral edge adjacent to the cup area (46) may be screwed into a female thread (27) in the jacket (20) and upon being screwed home presses against the cup body (40).

**4.** A hip cup as in one of the Claims 1 to 3,
characterized in that the female thread (25) for the cup body (40) and the female thread (27) for the threaded ring (72) are on opposite hands.

**5.** A hip cup as in one of the preceding Claims,
characterized in that the jacket (20) is closed at the apex of the cup body (40) and has an outwards directed polygonal portion (30) which may be inserted in a corresponding polygonal opening (10) at the apex of the outer cup (2).

**6.** A hip cup as in one of the preceding Claims,
characterized in that the fasteners (62; 82) include a circlip (62) which may be inserted removably in a circumferential groove (8) in the outer cup (2) and projects radially over the insert (50).

**7.** A hip cup as in Claim 6,
characterized in that the circlip (62) projects radially over the peripheral edge (28) of the jacket (20) and the threaded ring (72) between the jacket (20) and the cup body (40).

**8.** A hip cup as in one of the Claims 1 to 5,
characterized in that the fasteners (62, 82) include a locking ring (82) with a male thread (84), that the outer cup (2) has a corresponding locking thread (18) lying on the inside, which starts from the peripheral edge (11) and projects above the insert (50) in the direction of the axis (80), and that the locking ring (82) is screwed into the locking thread (18) firmly against the insert (50).

9. A hip cup as in Claim 7,
characterized in that the locking ring (82) in its final position rests flat against the threaded ring (72) of the insert (50).

10. A hip cup as in Claim 8,
characterized in that the locking ring (82) exhibits a female thread (86) which upon screwing the locking ring (82) into the outer cup (2) exhibits a circumferential lip directed radially outwards and presses into the turns of thread, permanently deformed.

11. A hip cup as in Claim 10,
characterized in that the threads on the outside (84) and inside (86) of the locking ring are on opposite hands.

12. A hip cup as in one of the preceding Claims,
characterized in that the shape of the inside of the outer cup (2) and the shape of the outside of the jacket (20) have each a conical portion (12 and 22 respectively) which continues at its wide end into an open cylindrical portion (14 and 24 respectively).

13. A hip cup as in Claim 12,
characterized in that the outer cup (2) at the narrow end of the conical portion (12) continues into the polygonal opening (10), and that the corresponding polydon (30) on the jacket (20) is seated at the narrow end of the conical portion (12) on a plane peak area.

14. A hip cup as in one of the Claims 12 or 13,
characterized in that the female thread (24) on the jacket (20) is arranged on the conical portion (22), running round the axis (80) of the cane, and that the bearing area (42) of the cup body (40) exhibits a corresponding conical portion (42a) which carries the corresponding male thread (44).

15. A hip cup as in one of the preceding Claims,
characterized in that the insert (50) consisting of the jacket (20) and cup body (40) is arranged in the outer cup (2) without clamping.

16. A hip cup as in one of the preceding Claims,
characterized in that the wall of the jacket (20) is of constant thickness.

**Revendications**

1. Cotyle de hanche (1) pour une endoprothèse de l'articulation de la hanche, comportant une coupelle externe métallique (2) et comportant un corps de coupelle (40) en matière plastique dans la coupelle externe (2), qui possède, du côté frontal, une surface de coupelle (46) à découvert, et, dans la coupelle externe (2), une surface d'appui de révolution (12), la forme intérieure de la coupelle externe (2) et la surface d'appui (42) du corps de coupelle (40) se rétrécissant vers le sommet, caractérisé par le fait que la surface d'appui du corps de coupelle (40) est entourée étroitement, au moyen d'au moins un élément de tension (72) sous l'effet de la pression superficielle, par une chemise métallique (20), l'élément de tension (72) étant en prise, d'une part, avec le corps de coupelle (40) et, d'autre part, avec la chemise métallique (20), et que l'insert (50), formé par le corps de coupelle (40) et la chemise (20), peut être étroitement ancré dans la coupelle externe (2) avec au moins un élément de fixation (62 ; 82).

2. Cotyle de hanche selon la revendication 1, caractérisé par le fait que le corps de coupelle (40) possède, sur sa surface d'appui (42), un filetage externe (44) qui est susceptible d'être vissé à bloc dans un filetage interne correspondant (25) de la chemise (20).

3. Cotyle de hanche selon l'une quelconque des revendications 1 et 2, caractérisé par le fait que les éléments de tension comprenant une bague filetée (72), qui est susceptible d'être vissée, sur le bord périphérique voisin de la surface de coupelle (46), dans un filetage interne (27) de la chemise (20) et qui appuie, lors de son vissage, contre le corps de coupelle (40).

4. Cotyle de hanche selon l'une quelconque des revendications 1 à 3, caractérisé par le fait que le filetage interne (25) pour le corps de coupelle (40) et le filetage interne (27) pour la bague filetée (72) sont opposés.

5. Cotyle de hanche selon l'une quelconque des revendications précédentes, caractérisé par le fait que la chemise (20) est fermée sur le sommet du corps de coupelle (40) et possède un polygone (30) orienté vers l'extérieur qui est enfichable dans une ouverture polygonale correspondante (10) sur le sommet de la coupelle externe (2).

6. Cotyle de hanche selon l'une quelconque des revendications précédentes, caractérisé par le fait que les éléments de fixation (62 ; 82) comprennent un jonc (62) qui est susceptible d'être introduit de façon amovible dans une rainure périphérique (8) de la coupelle externe (2) et qui fait saillie radialement de l'insert (50).

7. Cotyle de hanche selon la revendication 6, caractérisé par le fait que le jonc (62) dépasse radialement du bord périphérique (28) de la chemise (20) et de la bague filetée (72) entre la chemise (20) et le corps de coupelle (40).

8. Cotyle de hanche selon l'une quelconque des revendications 1 à 5, caractérisé par le fait que les éléments de fixation (62 : 82) comprennent une bague de sécurité (82) présentant un filetage externe (84), que la coupelle externe (2) possède, à partir du bord périphérique (11), un filetage de sécurité (18) intérieur correspondant, lequel fait saillie de l'insert (50) dans la direction de l'axe (80), et que la bague de sécurité (82) est suceptible d'être vissée à bloc dans le filetage de sécurité (18) contre l'insert (50).

9. Cotyle de hanche selon la revendication 7, caractérisé par le fait que la bague de sécurité (82) s'étend, dans sa position d'extrémité, pour venir par sa surface contre la bague filetée (72) de l'insert (50).

10. Cotyle de hanche selon la revendication 8, caractérisé par le fait que la bague de sécurité (82) présente un filetage interne (86), lequel présente, lors du vissage de la bague de sécurité (82) dans la coupelle externe (2), une lèvre périphérique orientée radialement vers l'extérieur et vient en appui sous l'effet de la déformation permanente dans les pas de vis.

11. Cotyle de hanche selon la revendication 10, caractérisé par le fait que le filetage externe (84) et le filetage interne (86) de la bague de sécurité sont opposés.

12. Cotyle de hanche selon l'une quelconque des revendications précédentes, caractérisé par le fait que la forme interne de la coupelle externe (2) et la forme externe de la chemise (20) possèdent une section conique (12 ou 22), qui, sur leur extrémité large, se transforme en une section cylindrique ouverte (14 ou 24).

13. Cotyle de hanche selon la revendication 12, caractérisé par le fait que la coupelle externe (2), sur l'extrémité étroite de la section conique (12) se transforme en l'ouverture polygonale (10), et que le polygone correspondant (30) de la chemise (20) est en appui, à l'extrémité étroite de la section conique (22), sur une surface de sommet plane.

14. Cotyle de hanche selon l'une quelconque des revendications 12 et 13, caractérisé par le fait que le filetage interne (25) de la chemise (20) est disposé autour de l'axe du cône (80) de façon circulaire en cône (22), et que la surface d'appui (42) du corps de coupelle (40) présente une section conique correspondante (42a), laquelle porte le filetage externe correspondant (44).

15. Cotyle de hanche selon l'une quelconque des revendications précédentes, caractérisé par le fait que l'insert (50) formé par la chemise (20) et le corps de coupelle (40) est disposé sans serrage dans la coupelle externe (2).

16. Cotyle de hanche selon l'une quelconque des revendications précédentes, caractérisé par le fait que la chemise (20) possède une épaisseur de paroi constante.

FIG. 1

FIG. 2

FIG. 3

Fig. 4

Fig. 5